# EUROPEAN PATENT APPLICATION

(11) **EP 4 803 086 A1**
(43) Date of publication of application: **09.09.2026**
(21) Application number: 24885572.8
(22) Date of filing: 24.10.2024
(51) Int. Cl.: A61K 31/685, A61K 35/56, A61K 35/57, A61K 35/618, A61P 3/06, A61P 3/10, C07K 14/575

(54) **ADIPONECTIN ENHANCER, ORAL COMPOSITION FOR ENHANCING ADIPONECTIN, AND USE OF PLASMALOGEN**

(30) Priority: 31.10.2023 JP 2023186111
(71) Applicant: Institute of Rheological Function of Food Co., Ltd., Kasuya-gun, Fukuoka 811-2501 (JP)
(72) Inventor: FUJINO Takehiko, Fukuoka-shi, Fukuoka 813-0043 (JP); MAWATARI Shiro, Fukuoka-shi, Fukuoka 813-0016 (JP); NIWASE Shamim, Fukuoka-shi, Fukuoka 810-0054 (JP); HONSHO Masanori, Fukuoka-shi, Fukuoka 810-0064 (JP)
(74) Representative: V.O.
(86) International application number: PCT/JP2024/037865
(87) International publication number: WO 2025/094799

(57) **Abstract**

An adiponectin enhancer includes a plasmalogen as an effective component.

## Description

### Technical Field

The present disclosure relates to an adiponectin enhancer, an oral composition for enhancing adiponectin, and use of plasmalogen.

### Background Art

Adiponectin is a protein secreted by fat cells. Various functions of adiponectin are known, such as activating PPARα that promotes burning of fatty acids in skeletal muscle and activating AMP kinase that promotes burning of fatty acids and uptake of glucose. Since adiponectin improves insulin resistance in skeletal muscle and in liver, drugs and the like that increase the adiponectin expression have been studied.

Patent Literature 1 discloses an adiponectin enhancer including a hop as an effective component. Non Patent Literature 1 discloses that thiazolidinedione (TZD) increases adiponectin in serum. Non Patent Literature 2 discloses that metformin increases adiponectin in serum. Non Patent Literature 3 discloses that omega-3 fatty acids increases adiponectin in serum. Non Patent Literature 4 discloses that resveratrol increases adiponectin in serum.

### Citation List

### Patent Literature

Patent Literature 1: Unexamined Japanese Patent Application Publication No. 2006-306800

### Non Patent Literature

Non Patent Literature 1: Norikazu Maeda, and 14 others, "PPARγ Ligands Increase Expression and Plasma Concentrations of Adiponectin, an Adipose-Derived Protein", DIABETES, 2001, 50(9), 2094-2099
Non Patent Literature 2: N Adamia, and four others, "Effect of metformin therapy on plasma adiponectin and leptin levels in obese and insulin resistant postmenopausal females with type 2 diabetes", Georgian Med News., 2007, 145, 52-55
Non Patent Literature 3: Mehdi Bahreni, and three others, "The Effect of Omega-3 on Circulating Adiponectin in Adults With Type 2 Diabetes Mellitus: A Systematic Review and Meta-Analysis of Randomized Controlled Trials", Can J Diabetes, 2018, 42, 553-559
Non Patent Literature 4: Cintia dos Santos Costa, and seven others, "Resveratrol Upregulated SIRT1, FOXO1, and Adiponectin and Downregulated PPARγ1-3 mRNA Expression in Human Visceral Adipocytes", OBES SURG, 2011, 21, 356-361

### Summary of Invention

### Technical Problem

As described above, various substances have been examined as drugs that enhance adiponectin. There is a need for additional drug candidates capable of enhancing adiponectin to ensure a diversity of such drugs available for clinical use.

The present disclosure is made in view of the aforementioned circumstances, and an objective of the present disclosure is to provide an adiponectin enhancer having an adiponectin enhancing effect, and an oral composition for enhancing adiponectin. A further objective of the present disclosure is to provide use of plasmalogen for manufacturing the adiponectin enhancer or the oral composition for enhancing adiponectin.

### Solution to Problem

An adiponectin enhancer according to a first aspect of the present disclosure includes a plasmalogen as an effective component.

An oral composition for enhancing adiponectin according to a second aspect of the present disclosure includes a plasmalogen as an effective component.

The plasmalogen may be extracted from an animal tissue.

The animal tissue may be an animal tissue selected from the group consisting of shellfish, sea squirts, and birds.

The animal tissue may be a tissue of a scallop.

Use according to a third aspect of the present disclosure is use of plasmalogen for manufacturing the adiponectin enhancer or the oral composition for enhancing adiponectin.

### Advantageous Effects of Invention

According to the present disclosure, an adiponectin enhancer having an adiponectin enhancing effect, and an oral composition for enhancing adiponectin are provided. According to the present disclosure, use of plasmalogen for manufacturing the adiponectin enhancer or the oral composition for enhancing adiponectin is also provided.

### Brief Description of Drawings

FIG. 1 is a diagram showing results of the Western blotting performed on rats in an example;
FIG. 2A is a diagram showing microscopic images of small intestinal tissue in immunohistochemical staining from rats in an example;
FIG. 2B is a diagram showing the relative number of adiponectin expressing cells quantified from the microscopic image shown in FIG. 2A;
FIG. 3 is a diagram showing the relative amount of adiponectin in serum of rats in an example;
FIG. 4A is a diagram showing microscopic images of small intestinal tissue in immunohistochemical staining relating to mice in an example;
FIG. 4B is a diagram showing the relative number of adiponectin expressing cells quantified from the microscopic images shown in FIG. 4A;
FIG. 5 is a diagram showing the relative expression level of RNA (AdipoQ) encoding adiponectin in cultured mouse intestinal epithelial tissue according to an example; and
FIG. 6 is a diagram showing the relative amount of adiponectin in culture supernatant according to an example.

### Description of Embodiments

Embodiments according to the present disclosure are described below with reference to the drawings. In this regard, the present disclosure is not limited by the below-described embodiments or the drawings. Note that, in the below-described embodiments, the expression, "have", "include", or "contain" encompasses the meaning of "composed of" or "be constituted by".

The adiponectin enhancer according to the present embodiment includes plasmalogen as an effective component. Plasmalogen belongs to a subclass specific to glycerophospholipids characterized by the presence of a vinyl ether bond at the sn-1 position and an ester bond at the sn-2 position of the glycerol backbone. Plasmalogen is present at high concentration in the cell membrane of a large number of mammalian tissues.

The plasmalogen included in the adiponectin enhancer is not limited as long as it is generally classified as plasmalogens. Examples of the plasmalogens include Pls, PlsCho, inositol-type plasmalogen, and serine-type plasmalogen. Pls has a structure in which -CH₂CH₂NH₂ is bound to the oxygen atom that is bound to the phosphorus at the sn-3 position of the glycerol backbone. PlsCho has a structure in which -CH₂CH₂N(CH₃)₃ is bound to the oxygen atom that is bound to the phosphorus at the sn-3 position of the glycerol backbone.

Preferably, the plasmalogen is extracted from animal tissue. The animal tissue is not particularly limited as long as it is animal tissue containing plasmalogen. Examples of the animal tissue include tissues of animals selected from the group consisting of shellfish, sea squirts, and birds. More specifically, tissues of aquatic animals such as shellfish, sea squirts, sea cucumbers, salmon, Pacific saury, and skipjack tuna are preferred as the animal tissue. Preferably, the animal tissue is a tissue of shellfish. An edible part of the animal tissue is preferred. The animal tissue may be a cut tissue, or may be a tissue that has been pulverized to extract plasmalogen more efficiently.

Examples of the shellfish include bivalves or snails, such as scallops, mussels, and abalones. Scallops are particularly preferred as the shellfish. Scallops are edible bivalves belonging to the family Pectinidae, and their examples include scallops belonging to the genus *Mizuhopecten* or the genus *Pecten*. Specific examples of the shellfish include a scallop harvested in Japan (*Mizuhopecten yessoensis*) and an European scallop harvested in Europe (*Pecten maximus* (Linnaeus)). Examples of edible parts of the scallops include adductor muscle and fringe.

Sea squirts are edible chordates belonging to the family Pyuridae, and their examples include those belonging to the genus *Halocynthia* or *Halocynthia aurantium*. Specific examples of the sea squirts include maboya (*Halocynthia roretzi*) and akaboya (*Halocynthia aurantium*). Examples of edible parts of the sea squirts include the flesh portion (fascial body).

Examples of the birds include edible birds. Examples of the birds include chickens, Silkies, and ducks. As the bird tissue, breast meat is preferred because it abundantly contains plasmalogen.

Plasmalogen can be extracted using an organic solvent or an aqueous organic solvent, and is preferably extracted using enzyme treatment in combination. Examples of the extraction method for plasmalogen include an ethanol extraction method and a hexane extraction method.

In the ethanol extraction method, the animal tissue may be exposed to an extraction liquid containing ethanol. The extraction liquid containing ethanol used in the extraction step may be aqueous ethanol or a mixture of ethanol and another organic solvent. The ethanol concentration in the extraction liquid is not less than 50% by mass, preferably not less than 80% by mass, more preferably not less than 95% by mass, or may be 100% by mass. The amount of the ethanol used is, for example, preferably 100 to 3000 mL, more preferably 200 to 2000 mL, still more preferably 250 to 1000 mL per 100 g of the animal tissue. The extraction using ethanol may be carried out a plurality of times.

Preferably, the animal tissue exposed to the extraction liquid containing ethanol is animal tissue treated with a protease. The protease is, for example, a protease derived from a filamentous fungus of the genus *Aspergillus*, the genus *Rhizopus*, or the like; a protease derived from a bacterium of the genus *Bacillus* or the like; or a protease extracted from a plant such as papaya or pineapple. As the protease, a commercially available protease may be used. In particular, a protease derived from a filamentous fungus is preferred, and a protease derived from koji mold is more preferred. Since plasmalogen is unstable under acidic and basic conditions, a neutral protease is preferred.

The amount of the protease used is, for example, 0.1 to 10.0 g, preferably 0.2 to 8.0 g, more preferably 0.3 to 5.0 g for 100 g of the animal tissue.

After the extraction, solids are removed and the extraction liquid is collected, followed, when necessary, by drying to solidify the extract, to obtain an extract containing plasmalogen. The extract may be subjected to a concentration treatment, thereby concentrating the plasmalogen.

In the case of a hexane extraction method, total lipids contained in an extract obtained by an ethanol extraction method are subjected to extraction treatment with a mixed solvent of n-hexane and a water-soluble ketone solvent, to achieve separation into an insoluble fraction and a soluble fraction. The water-soluble ketone solvent is, for example, acetone, methyl ethyl ketone, or a combination thereof. The water-soluble ketone solvent is preferably acetone. In cases where a mixture of n-hexane and acetone is used as the mixed solvent, their volume ratio is, for example, 4:6 to 6:4, preferably 4.5:5.5 to 5.5:4.5. The amount of the mixed solvent used is, for example, 10 to 30 mL per 1 g (dry mass) of total lipids.

After drying the soluble fraction obtained by the extraction treatment with the mixed solvent, plasmalogen can be separated and recovered by performing extraction treatment with a water-soluble ketone solvent.

The content of plasmalogen in the adiponectin enhancer according to the present embodiment is appropriately adjusted. For example, the adiponectin enhancer may contain, as an effective component, 0.000001 to 99.9% by mass, 0.00001 to 99.8% by mass, 0.0001 to 99.7% by mass, 0.001 to 99.6% by mass, 0.01 to 99.5% by mass, 0.1 to 99% by mass, 0.5 to 60% by mass, 1 to 50% by mass, or 1 to 2% by mass plasmalogen.

Examples of the subject to which the adiponectin enhancer according to the present embodiment is administered include cells, biological tissue, organisms, and animals. The adiponectin enhancer as a pharmaceutical is administered to humans or animals. Preferred examples of the animals include mammals, more specifically, dogs, cats, cows, pigs, horses, sheep, and deer.

The route of administration of the adiponectin enhancer according to the present embodiment to humans, or the like, is not particularly limited. The adiponectin enhancer is preferably used as an external preparation, an injection, or an oral preparation. The adiponectin enhancer may contain, for example, the plasmalogen and a pharmaceutically acceptable carrier. The term "pharmaceutically acceptable carrier" refers to any of various types of organic carrier substances and inorganic carrier substances that are used as materials for pharmaceutical preparations. The pharmaceutically acceptable carriers are, for example, excipients, binders, disintegrants, lubricants, stabilizers, and flavoring and deodorizing agents in solid preparations, or solvents, solubilizing agents, suspending agents, tonicity agents, buffering agents, and flavoring and deodorizing agents in liquid preparations. An additive such as antiseptic agents, antioxidants, coloring agents, or sweeteners may also be optionally blended.

Examples of the excipients include lactose, white sugar, D-mannitol, D-sorbitol, starch, α-starch, dextrin, crystalline cellulose, low-substituted hydroxypropyl cellulose, sodium carboxymethylcellulose, gum arabic, pullulan, light anhydrous silicic acid, synthetic aluminum silicate, magnesium aluminometasilicate, xylitol, sorbitol, and erythritol.

Examples of the binders include, in addition to the excipients above, α-starch, sucrose, gelatin, gum arabic, methyl cellulose, carboxymethyl cellulose, sodium carboxymethylcellulose, crystalline cellulose, white sugar, D-mannitol, trehalose, dextrin, pullulan, hydroxypropyl cellulose, hydroxypropylmethylcellulose, and polyvinylpyrrolidone.

Examples of the disintegrants include, in addition to the excipients above, lactose, white sugar, starch, carboxymethyl cellulose, carboxymethyl cellulose calcium, croscarmellose sodium, carboxymethyl starch sodium, low-substituted hydroxypropyl cellulose, light anhydrous silicic acid, and calcium carbonate.

Examples of the lubricants include magnesium stearate, calcium stearate, talc, colloidal silica, and polyethylene glycol.

Examples of the stabilizers include paraoxybenzoic acid esters such as methylparaben and propylparaben; alcohols such as chlorobutanol, benzyl alcohol, and phenylethyl alcohol; benzalkonium chloride; acetic anhydride; and sorbic acid. Examples of the flavoring and deodorizing agents include sweeteners, acidulants, and flavors.

Examples of the solvents include water for injection, physiological saline, Ringer solution, alcohol, propylene glycol, polyethylene glycol, sesame oil, corn oil, olive oil, and cottonseed oil. Examples of the solubilizing agents include polyethylene glycol, propylene glycol, D-mannitol, trehalose, benzyl benzoate, ethanol, tris(hydroxymethyl)aminomethane, cholesterol, triethanolamine, sodium carbonate, sodium citrate, sodium salicylate, and sodium acetate.

Examples of the suspending agents include surfactants such as stearyltriethanolamine, sodium lauryl sulfate, lauryl aminopropionic acid, lecithin, benzalkonium chloride, benzethonium chloride, and glycerol monostearate; hydrophilic polymers such as polyvinyl alcohol, polyvinylpyrrolidone, sodium carboxymethylcellulose, methyl cellulose, hydroxymethylcellulose, hydroxyethylcellulose, and hydroxypropyl cellulose; polysorbates; and polyoxyethylene hydrogenated castor oils.

Examples of the tonicity agents include sodium chloride, glycerin, D-mannitol, D-sorbitol, glucose, xylitol, and fructose. Examples of the buffering agents include buffer solutions such as phosphate, acetate, carbonate, and citrate buffers.

Examples of the antiseptic agents include paraoxybenzoic acid esters, chlorobutanol, benzyl alcohol, phenethyl alcohol, dehydroacetic acid, and sorbic acid. Examples of the antioxidants include sulfite and ascorbic acid. Examples of the coloring agents include water-soluble tar dyes, lake pigments, and natural colorants. Examples of the sweeteners include sodium saccharin, dipotassium glycyrrhizinate, aspartame, and stevia.

The adiponectin enhancer according to the present embodiment is produced by a known method, and provided in the form of liquids, creams, ointments, pills, granules, fine granules, powders, tablets, capsules, or the like.

The dose of the adiponectin enhancer according to the present embodiment is determined as appropriate depending on, for example, the age, body weight, and symptom of a human or animal to which the anti-aging agent is administered. The adiponectin enhancer is administered in such an amount that an effective amount of the plasmalogen is contained therein. The effective amount is the amount of the plasmalogen required for obtaining a desired result, and is the amount required for delaying, inhibiting, preventing, or reversing the progression of, or for curing of, the condition to be treated or handled.

The dose of the adiponectin enhancer according to the present embodiment is, for example, 0.000001 µg or more per day for an adult, preferably 1 µg or more or 500 µg or more, more preferably 1000 µg or more. The upper limit is, for example, 20 mg per day, preferably 10 mg. The adiponectin enhancer can be administered once or multiple times per day. Further, the adiponectin enhancer may be administered in any of various types of dosing frequencies, such as, for example, every other day, once a week, every other week, and once a month. If necessary, a dose other than the above-described range can also be used.

The plasmalogen that is an effective component in the adiponectin enhancer according to the present embodiment exhibits an effect of increasing the adiponectin expression in intestinal cells and the amount of adiponectin in serum as shown in the Examples below.

The adiponectin enhancer according to the present embodiment may be used as a reagent for the purpose of enhancing the adiponectin expression in *in vitro* and *in vivo* experiments.

In another aspect of the present embodiment, an oral composition for enhancing adiponectin is provided. Specific examples of such an oral composition include supplements, food and beverage compositions, functional foods, and food additives.

The forms of the supplements are not limited, and may be any of tablets, powders, granules, capsules, sugar-coated tablets, film formulations, troches, chewable formulations, solutions, emulsions, suspensions, and the like. The supplements may include any components that are normally used for supplements.

"Functional food" means a food or beverage consumed for the purpose of maintaining health, and examples of the functional foods include: foods for specified health uses, foods with function claims, and foods with nutrient function claims that are foods with health claims; health foods; dietary supplements; and the like. The functional foods are preferably foods for specified health uses or foods with nutrient function claims that are foods with health claims. In cases of commercialization as a functional food, the oral composition for enhancing adiponectin may include various additives for use in foods, such as colorants, preservatives, thickening stabilizers, antioxidants, bleaching agents, antibacterial and antifungal agents, acidulants, sweeteners, seasonings, emulsifiers, fortifiers, agents for production, flavors, and the like.

The functional food may be either a food or beverage, and is not limited as long as the functional food can be orally taken. Examples of the forms of the functional food include beverages, confectionery, processed cereal products, kneaded products, dairy products, and seasonings. Examples of the beverages include nutritional drinks, soft drinks, black tea, and green tea. Examples of the confectionery include candies, cookies, tablets, chewing gums, and jellies. Examples of the processed cereal products include bread, cooked rice, and biscuits. Examples of the kneaded products include sausage, ham, and *kamaboko*, which is boiled fish paste. Examples of the dairy products include butter and yogurt.

The oral composition for enhancing adiponectin may be added to foods as food additives. In such a case, examples of the food additives may include pastes, gel-like agents, powders, liquids, suspensions, emulsions, and granules, from the viewpoint of easily adding the food additive to foods.

The oral composition for enhancing adiponectin according to the present embodiment may contain water, vitamins, minerals, organic acids, organic bases, fruit juices, flavors, functional components, food additives, and the like, as long as the oral composition for enhancing adiponectin maintains effectiveness related to adiponectin enhancement. The oral composition for enhancing adiponectin may be produced by a known method in which components other than the plasmalogen are added, as necessary.

The oral composition for enhancing adiponectin may be divided into one or more containers such that the daily intake is in accordance with the above-described amount of intake. In such a case, the oral composition for enhancing adiponectin is preferably contained in each container in an amount corresponding to the daily intake.

From the viewpoint of the fact that the oral composition for enhancing adiponectin contains the plasmalogen and is used for adiponectin enhancement, the oral composition for enhancing adiponectin is provided in the forms of products that are distinguishable from other products. For example, at least one of the product packaging, instructions, and advertisements for the oral composition for enhancing adiponectin indicates that the oral composition for enhancing adiponectin has an adiponectin-enhancing function.

When the oral composition for enhancing adiponectin according to the present embodiment is provided as a food and beverage composition, the oral composition for enhancing adiponectin may be offered or sold as a food or beverage with an indication of the application for adiponectin enhancement. The application includes health purposes. The act of "indication" includes all acts that inform consumers of the aforementioned application. Any expression that evokes or suggests the aforementioned application is considered an act of "indication", regardless of the purpose, content, object, medium, and the like of the indication.

The "indication" is preferably made in a manner that allows the consumers to directly recognize the aforementioned application. Specifically, this includes acts such as an act of transferring, delivering, exhibiting for transfer or delivery, or importing food and beverage products or product packaging with the aforementioned application described; an act of exhibiting or distributing advertisements, price lists, or transaction documents relating to the products with the aforementioned application described, or providing information of the content with the aforementioned application described via electromagnetic means such as the Internet.

The "indication" may include indications as health foods, functional foods, enteral nutritional foods, foods for special uses, foods with health claims, foods for specified health uses, foods with nutrient function claims, foods with function claims, quasi-drugs, and the like. Among these, particularly, indications approved under systems for foods for specified health uses, foods with nutrient function claims, or foods with function claims, or similar systems are included. Specifically, examples of the indications include an indication as foods for specified health uses, an indication as conditional foods for specified health uses, an indication of effects on body structure or function, an indication for disease risk reduction, and an indication of functionality based on scientific evidence. More specifically, typical examples thereof include an indication as foods for specified health uses, in particular, an indication of health purposes, and similar indications.

Furthermore, in another aspect of the present embodiment, use of plasmalogen for manufacturing the adiponectin enhancer or the oral composition for enhancing adiponectin is also provided. In yet another aspect of the present embodiment, a method of enhancing adiponectin including a step of administering the plasmalogen to a subject is provided. In still another aspect of the present embodiment, the plasmalogen for the use in adiponectin enhancement is provided. The adiponectin enhancement includes acts of preventing, suppressing, inhibiting, and restoring the decrease in the expression level of adiponectin.

The present disclosure is more specifically described with reference to the following Examples, but the present disclosure is not limited to the Examples.

### Examples

### [Preparation of Plasmalogens (sPls)]

Plasmalogens (sPls) were prepared from a scallop (*Mizuhopecten yessoensis*) by the following method. The sPls were extracted from fresh scallop mantle using hexane by the method described in Japanese Patent No. 7304643. In extraction of plasmalogens from the scallop, first, Kokulase P (manufactured by Mitsubishi-Chemical Foods Corporation) and Phospholipase A1 (PLA1, manufactured by Mitsubishi-Chemical Foods Corporation) were added to the fresh scallop mantle, and the mixture was decomposed for 1 hour. Subsequently, hexane/isopropanol was added thereto, and the resulting mixture was stirred and left. Thereafter the supernatant was subjected to suction filtration. An aqueous sodium sulfate solution was added to the filtrate and the mixture was well mixed, and an upper layer was dried to solidification using a rotary evaporator. 40 mL of acetone cooled to 4°C was added to the dried material and the mixture was mixed. Using a centrifuge, the mixture was centrifuged for 10 minutes at 3000 rpm at 4°C. The supernatant was discarded, a precipitate was collected, and the precipitate was dried overnight in a desiccator. Following the disclosure of Japanese Patent No. 5430566, the precipitate was separated by HPLC using a mobile phase solvent, and only ethanolamine-type plasmalogens were collected and, which results in the sPls.

### [Study of adiponectin expression in intestinal cells of rats]

The sPls were administered to 12-week-old Wistar rats (5 rats per group, n = 5) by drinking water at a dose of 1 mg/kg for 14 days (sPls group). Blood was collected, and after intracardiac perfusion with chilled phosphate-buffered saline (PBS), small intestinal tissue washed with chilled PBS was collected from each rat. Proteins were extracted from a portion of the small intestinal tissue for Western blotting. The remaining small intestinal tissue was subjected to immunohistochemical staining. Antibodies used for Western blotting or immunohistochemical staining were an anti-adiponectin antibody (manufactured by Cell Signaling Technology) and β-Actin (manufactured by Santa Cruz). DAPI was used to stain the cell nuclei.

Serum was separated from blood, and adiponectin in the serum was quantified by enzyme-linked immunosorbent assay (ELISA) (manufactured by Invitrogen, #A44838).

### [Results]

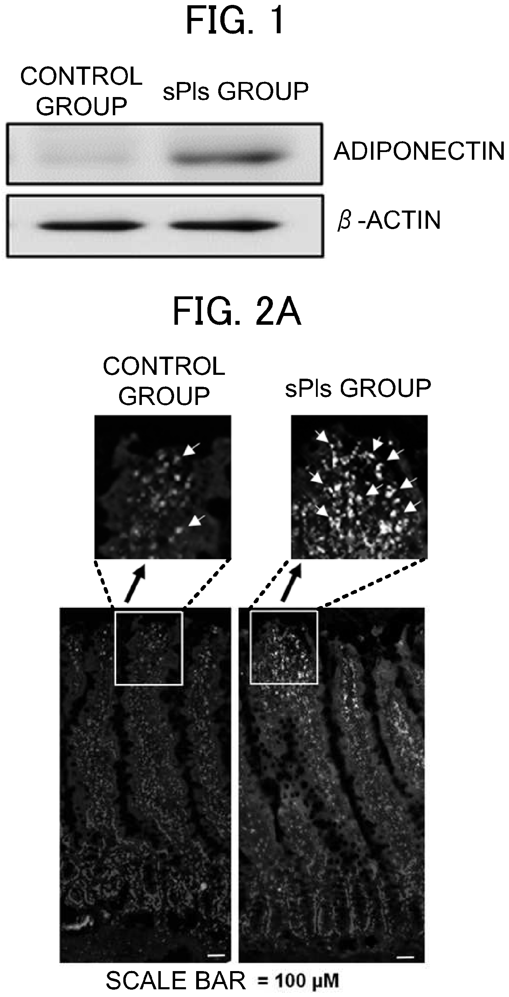

FIG. 1 shows results of Western blotting comparing a control group not administered the sPls and the sPls group. The relative change in expression level of adiponectin based on the Western blotting results was 2.84 fold (standard deviation: 0.21, p < 0.05).

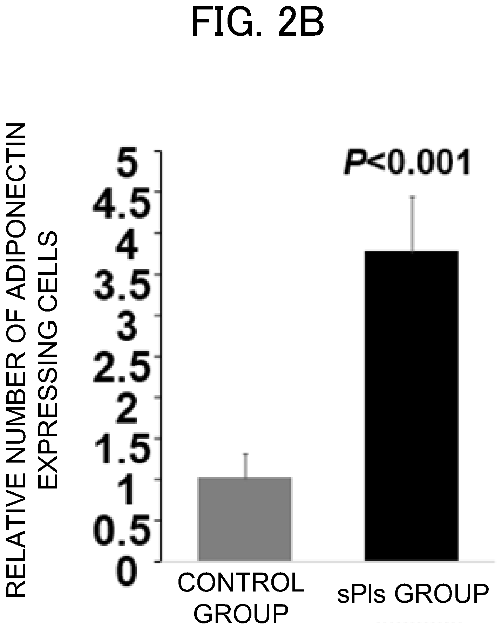

FIG. 2A shows microscopic images of small intestinal tissue subjected to immunohistochemical staining. Intake of the sPls increased the adiponectin expression in intestinal epithelial cells. The small arrows in FIG. 2A indicate adiponectin-expressing cells. The relative number of the adiponectin-expressing cells in FIG. 2A is shown in FIG. 2B. Intake of the sPls significantly increased intestinal epithelial cells expressing adiponectin.

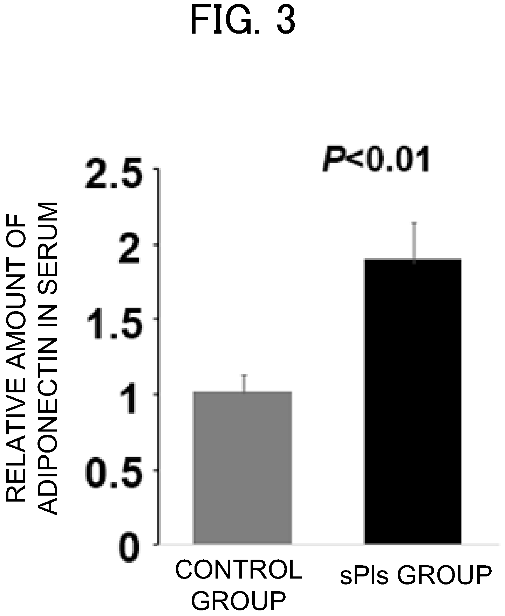

FIG. 3 shows the relative amount of adiponectin in serum quantified by ELISA. Intake of the sPls significantly increased adiponectin in serum.

### [Study of adiponectin expression in intestinal cells of mice]

The sPls were administered to 8-week-old male B6 mice (C57BL/6J) (5 mice per group, n = 5) by drinking water at a dose of 0.2 mg/kg for 14 days (sPls group). After intracardiac perfusion with chilled PBS, small intestinal tissue was collected from each mouse. The small intestinal tissue was subjected to immunohistochemical staining using anti-adiponectin antibodies (manufactured by Cell Signaling Technology). DAPI was used to stain the cell nuclei.

### [Results]

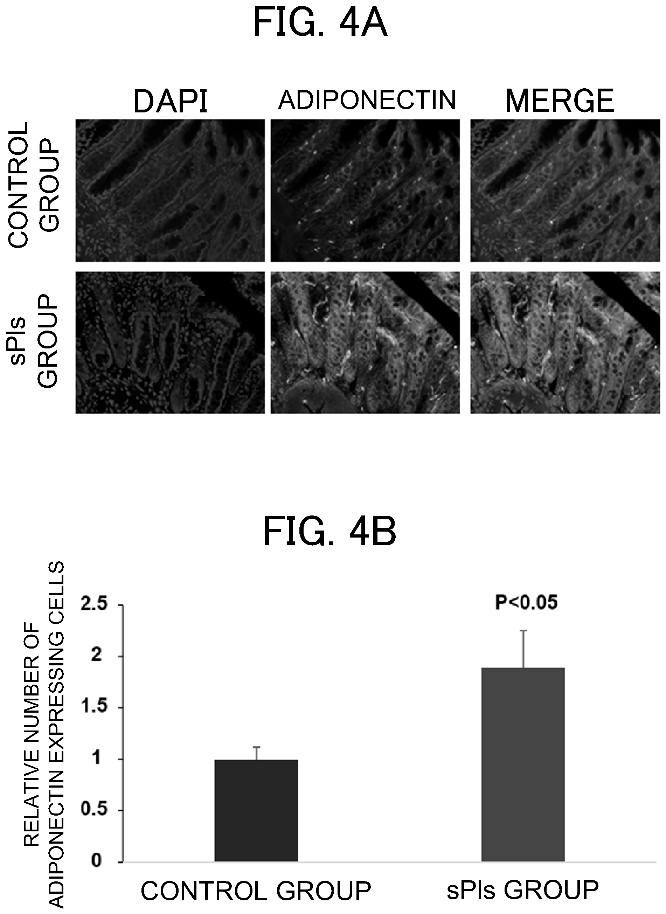

FIG. 4A shows microscopic images of small intestinal tissue subjected to immunohistochemical staining. Intake of the sPls increased the adiponectin expression compared with the control group not administered the sPls. The relative number of the adiponectin-expressing cells in FIG. 4A is shown in FIG. 4B. Intake of the sPls significantly increased intestinal epithelial cells expressing adiponectin. A p-value was calculated by the student-t test.

### [Study of adiponectin expression by plasmalogens in mouse intestinal epithelial tissue culture]

For 8-week old mature male B6 mice (C57BL/6J), after intracardiac perfusion with chilled PBS, small intestines were collected from each mouse. A portion of the small intestinal tissue washed with chilled PBS was excised, digested with Accutase at 37°C, and dissolved in DMEM. The dissolved tissue was filtered through a 100 µm mesh. 50 µl of the resulting tissue solution was mixed with 50 µl of Matrigel (manufactured by BD Biosciences) and cultured in DMEM supplemented with 10% fetal bovine serum (FBS). After 48 hours of culture, the sPls (5 µg/mL) were added to the medium and the culture was continued for an additional 16 hours (sPls group). The cultured tissue was collected and RNA was extracted. Proteins in culture supernatant were extracted.

Quantitative reverse transcription PCR was performed on the extracted RNA, and an expression level of AdipoQ was evaluated. The expression level of AdipoQ was normalized with Gapdh that is an endogenous control gene. A forward primer and a reverse primer having the base sequence of SEQ ID NO:1 and the base sequence of SEQ ID NO:2, respectively, were used for AdipoQ. A forward primer and a reverse primer having the base sequence of SEQ ID NO:3 and the base sequence of SEQ ID NO:4, respectively, were used for Gapdh.

The adiponectin in the extracted proteins was quantified by ELISA (manufactured by Invitrogen, #A44838).

### [Results]

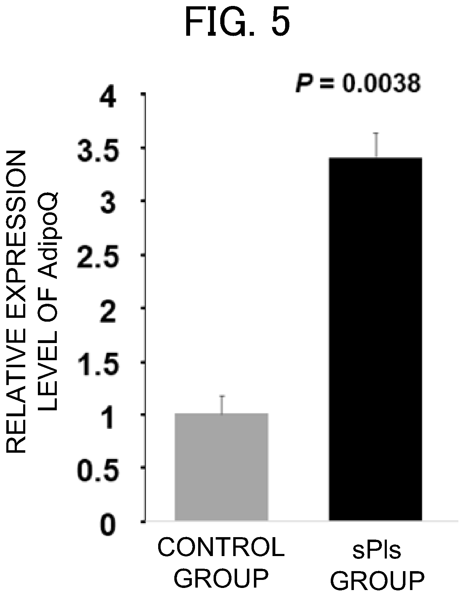

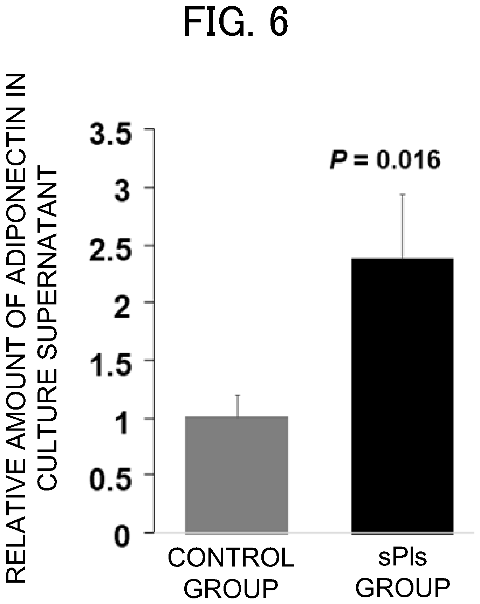

FIG. 5 shows the relative expression level of AdipoQ comparing a control group not treated with the sPls and the sPls group. In primary culture of mouse intestinal tissue, the expression level of AdipoQ was significantly increased (n = 8). FIG. 6 shows the relative amount of adiponectin in the culture supernatant quantified by ELISA. The treatment with the sPls significantly increased the adiponectin level in the culture supernatant (n = 8).

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2023-186111 filed on October 31, 2023, the entire disclosure of which is incorporated by reference herein.

### Industrial Applicability

The present disclosure is useful for adiponectin enhancer.

## Claims

1. An adiponectin enhancer, comprising:
a plasmalogen as an effective component.

2. An oral composition for enhancing adiponectin, the oral composition comprising:
a plasmalogen as an effective component.

3. The adiponectin enhancer according to claim 1 or the oral composition according to claim 2, wherein
the plasmalogen is extracted from an animal tissue.

4. The adiponectin enhancer or the oral composition according to claim 3, wherein
the animal tissue is an animal tissue selected from the group consisting of shellfish, sea squirts, and birds.

5. The adiponectin enhancer or the oral composition according to claim 3, wherein
the animal tissue is a tissue of a scallop.

6. Use of plasmalogen for manufacturing an adiponectin enhancer or an oral composition for enhancing adiponectin.
